(19) Europäisches Patentamt
European Patent Office
Office européen des brevets

(11) EP 4 001 405 A1

(12) # EUROPEAN PATENT APPLICATION
published in accordance with Art. 153(4) EPC

(43) Date of publication:
**25.05.2022 Bulletin 2022/21**

(21) Application number: **20839987.3**

(22) Date of filing: **05.06.2020**

(51) International Patent Classification (IPC):
***C12N 7/01*** *(2006.01)* ***C12N 15/57*** *(2006.01)*
***A61K 35/763*** *(2015.01)* ***A61K 35/768*** *(2015.01)*
***A61P 35/00*** *(2006.01)*

(52) Cooperative Patent Classification (CPC):
Y02A 50/30

(86) International application number:
**PCT/CN2020/094751**

(87) International publication number:
**WO 2021/008269 (21.01.2021 Gazette 2021/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.07.2019 CN 201910642804**

(71) Applicant: **Wu, Zetang**
**Hefei, Anhui 230088 (CN)**

(72) Inventor: **Wu, Zetang**
**Hefei, Anhui 230088 (CN)**

(74) Representative: **Hautier IP - MC/EP**
**1, Rue du Gabian**
**Le Thalès - 12 Etg - Bloc A**
**98000 Monaco (MC)**

(54) **ONCOLYTIC VIRUS AND APPLICATION THEREOF, AND DRUG FOR TREATING CANCER**

(57) Provided are an oncolytic virus and an application thereof, and a drug for treating a cancer. A first regulatory element and a second regulatory element are inserted into the genome of the oncolytic virus. The first regulatory element comprises a tumor-specific promoter and a first nucleic acid sequence, which is driven by the cancer cell specific promoter to express a specific protease in tumor cells; the second regulatory element comprises a second nucleic acid sequence for encoding an extracellular secretion signal peptide and a third nucleic acid sequence for encoding a specific cleavage site. The oncolytic virus can be replicated in tumor cells effectively to kill tumor cells while being safe to non-cancer cells.

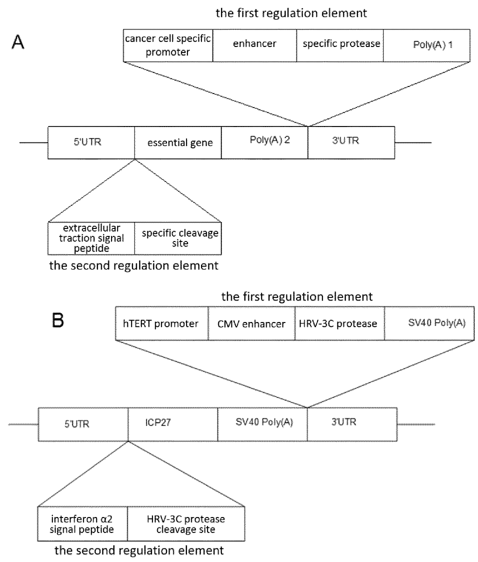


Figure. 1

EP 4 001 405 A1

## Description

### CROSS-REFERENCES TO RELATED APPLICATIONS

The present disclosure claims the priority of a Chinese patent application filed with the Chinese Patent Office on July 16, 2019, with the application number 2019106428044 and entitled "Oncolytic Virus and Application Thereof and Drug for Treating Cancer", the entire content of which is incorporated by reference in this application.

### TECHNICAL FIELD

The present disclosure relates to the technical field of cancer treatment, in particular, to an oncolytic virus and use thereof and drug for treating cancer.

### BACKGROUND ART

Cancer has become the number one killer affecting health. China is a region with a high incidence of cancer, especially lung cancer, gastric cancer, liver cancer and rectal cancer. According to statistics, in 2016 alone, there were 4.8 million new cancer patients nationwide, and 2.3 million patients died of various cancers. With the advancement of technology, various new treatments, especially immunotherapy, have been continuously put into clinical use. However, the demands for safe and effective therapies are far from being met. Therefore, it is imperative to develop new drugs for the treatment.

An article published in "Science" in 1991, proved genetically modified herpes simplex virus produced therapeutic benefits in treatment of malignant glioma. Since then, the development of oncolytic viruses to treat cancer has been attracting extensive attention. The basic concept of developing an oncolytic virus for treating cancer is to genetically modify a mildly pathogenic virus to achieve the tumor selectivity. Oncolytic viruses inhibit tumor growth by two mechanisms: oncolysis and anti-tumor immunity. Once an oncolytic virus enters a tumor, the virus replicates resulting in tumor cell death and lysis (oncolysis). And the cellular debris of the lyzed cells induces tumor-specific immunity, thus helping kill tumor cells in situ or attacking already metastasized tumor cells. Because of the unique features associated with oncolytic viruses, developing oncolytic viruses for treatment of cancer has been demonstrated to be a promising treatment strategy.

Up to date, new castle disease virus (NDV), herpes simplex virus (HSV), reovirus, vaccinia virus and adenovirus have been utilized to develop oncolytic viruses. Pre-clinical studies have demonstrated oncolytic viruses possess an excellent safety profile and are effective in treating various tumors in animals. However, the clinical performance of oncolytic viruses is generally poor. The caveats associated with currently available oncolytic viruses are attributed to the design strategies exploited for generation of oncolytic viruses. Currently, available oncolytic viruses were mainly developed by deleting one or more viral non-essential genes to confer the tumor selectivity to the virus. Viral non-essential genes are not required for the virus to grow in tissue culture. But the gene products of viral non-essential genes have been shown to directly or

Indirectly contribute to viral replication *in vivo*. Deleting non-essential genes from the viral genome allows the virus to selectively replicate in tumor cells, but the selectivity is achieved at the expenses of replication ability. That is why currently available oncolytic viruses generally perform poorly in clinic. In order to enhance the efficacy and broaden the application of oncolytic viruses in treatment of tumors, it is essential to maintain the integrity of the viral genome while not disrupting the temporal coordination of the viral gene expression. One option to achieve the goal is to confer tumor selectivity to the virus by adding exogenous sequences to regulate the expression of viral gene(s) such that the virus can only replicate in tumor cells.

Based on that concept, this disclosure is hereby provided

### SUMMARY

One aspect of the present disclosure provides an oncolytic virus, which selectively replicates in and kill tumor cells effectively while the virus is safe to non-tumor cells.

Another aspect of the present disclosure provides nucleic acid fragments for preparing the above-mentioned oncolytic virus.

Another aspect of the present disclosure provides an oncolytic virus containing the aforementioned nucleic acid fragments.

Another aspect of the present disclosure provides a method for preparing the above-mentioned oncolytic virus.

Another aspect of the present disclosure provides the use of the above-mentioned oncolytic virus in tumor treatment.

Another aspect of the present disclosure provides a drug for treating cancer.

In the first aspect, the present disclosure provides an oncolytic virus, in which a first regulatory element is inserted into the viral genome. The first regulatory element comprises a tumor-specific promoter and a first nucleic acid sequence, which is driven by the tumor-specific promoter to express specific protease in target cancer cells.

It should be noted that insertion of the first regulatory element is located between two genes of the oncolytic virus. It can be inserted between two essential genes, or between one essential gene and one non-essential gene.

A second regulatory element is also inserted into the genome of the oncolytic virus. The second regulatory element comprises: a second nucleic acid sequence for encoding an extracellular secretion signal peptide and a third nucleic acid sequence for encoding a specific cleavage site. The specific cleavage site is recognized and cleaved by the specific protease.

The second regulatory element is in frame inserted between the first and second codons of one essential gene of the oncolytic virus. Therefore, a fusion protein will be produced once the regulated essential gene is expressed.

When the oncolytic virus enters non-tumor cells such as normal cells, the specific protease thereof is not expressed. Therefore, the extracellular secretion signal peptide within the fusion protein will direct the fusion protein to secrete to the outside of the cells once the fusion protein is produced, resulting in a trace amount of or no gene product of the regulated viral gene remaining in the cells. As a result, there is no viral replication or the virus replicates extremely poor in the cells.

When the oncolytic virus enters tumor cells, the specific protease will be robustly expressed, and the fusion protein once produced will be recognized and cleaved by the specific protease, thus allowing the regulated viral protein to remain within the cells and play its function to support viral replication.

The recombinant oncolytic virus provided in the present disclosure does not delete any viral gene from the viral genome. Therefore, it can replicate in tumor cells with a similar efficacy to that observed for the wild-type virus, thus killing tumor cells effectively.

The genome structure of the oncolytic virus provided in the present disclosure is shown in Figure 1A.

In one or more embodiments of the present disclosure, the specific protease is selected from the group consisting of human rhinovirus 3C protease (HRV 3C protease), thrombin, factor Xa protease, tobacco etch virus protease (TEV protease) or recombinant PreScission protease.

Of course, it should be noted that the specific proteases related to the present disclosure is not limited to those described above. In some other embodiments, those skilled in the art can select suitable proteases as needed to be used in the present disclosure, which all fall into the protection scope of this disclosure.

Correspondingly, those skilled in the art can select suitable specific cleavage sites that can be recognized and cleaved by specific proteases as needed to apply to the present disclosure. For example,

When the specific protease is human rhinovirus 3C protease, the amino acid sequence of the specific cleavage site is: LEVLFQGP.

When the specific protease is thrombin, the amino acid sequence of the specific cleavage site is: LVPRGS.

When the specific protease is factor Xa protease, the amino acid sequence of the specific cleavage site is: IE/DGR (IEDGR or IDGR).

When the specific protease is tobacco etch virus protease, the sequence of the specific cleavage site is: ENLYFQG.

When the specific protease is recombinant PreScission protease, the sequence of the specific cleavage site is: LEVLFQGP.

In one or more embodiments of the present disclosure, the extracellular secretion signal peptide is selected from the group including interferon $\alpha 2$, interleukin 2, human serum albumin, human immunoglobulin heavy chain and luciferase extracellular secretion signal peptides.

Of course, it should be noted that the extracellular secretion signal peptides, which can be applied to the present disclosure, are not limited to those described above. In some other embodiments, those skilled in the art can select other appropriate extracellular secretion signal peptides as needed to apply to the present disclosure. As long as the extracellular secretion signal peptide can drive the protein fused with it to secrete to the outside of the cells, it should be within the protection scope of the present disclosure.

In one or more embodiments of the present disclosure, the first regulatory element further comprises an enhancer; the enhancer is inserted between the tumor-specific promoter and the specific protease encoding sequence. The enhancer is used to enhance the expression of the specific protease in tumor cells.

The present disclosure does not limit the enhancer types. Any enhancer that can enhance the expression of the downstream gene can be used in the present disclosure. In one or more embodiments, the enhancer is either CMV enhancer or SV40 enhancer. Of course, in other embodiments of the present disclosure, those skilled in the art can select other suitable enhancer as required, no matter which enhancer is selected, as long as its purpose is to be used for enhancing the expression of the specific protease in tumor cells, especially in tumor cells with low tumor-specific promoter activity, it falls within the scope of protection of the present disclosure.

In one or more embodiments of the present disclosure, the target tumor cells are lung cancer, liver cancer, breast cancer, gastric cancer, prostate cancer, brain tumor, human colon cancer, cervical cancer, and kidney cancer, ovarian cancer, head and neck cancer, melanoma, pancreatic cancer and esophageal cancer cells.

In one or more embodiments of the present disclosure, the tumor-specific promoter is selected from the group consisting of telomerase reverse transcriptase (hTERT), human epidermal growth factor receptor-2 (HER-2), E2F1, osteocalcin, carcinoembryonic antigen, survivin and ceruloplasmin promoters.

Of course, the tumor-specific promoters applied to the present disclosure are not limited to those mentioned above. In some other embodiments, those skilled in the art can select a suitable promoter that can specifically drive the expression of downstream genes in tumor cells as required. No matter which tumor-specific promoter is selected, it belongs to the protection scope of the present disclosure.

In one or more embodiments of the present disclosure, the oncolytic virus is selected from the group consisting of herpes simplex virus (such as herpes simplex virus type 1), adenovirus, vaccinia virus, newcastle disease virus, poliovirus, coxsackie virus, measles virus, mumps virus, vesicular stomatitis virus, and influenza virus.

It should be noted that virus types applied to the present disclosure are not limited to those mentioned above. In other embodiments, those skilled in the art can select a suitable oncolytic virus based on needed. But as long as it has the ability to infect and selectively tumor cells, no matter which virus is selected, it belongs to the protection scope of the present disclosure.

In addition, each virus contains several essential genes, and those skilled in the art can select genes other than the gene mentioned in the embodiments to make new oncolytic viruses by using the concepts provided in the present disclosure.

For example, when the oncolytic virus is herpes simplex virus, the essential gene is selected from the group consisting of envelope glycoprotein L, uracil DNA glycosylase, capsid protein, helicase proenzyme subunit, DNA replication initiation binding unwindase, derived protein of myristic acid, deoxyribonuclease, coat serine/threonine protein kinase, DNA packaging terminase subunit 1, coat protein UL16, DNA packaging protein UL17, capsid triplex subunit 2, major capsid protein, envelope protein UL20, nucleoprotein UL24, DNA packaging protein UL25, capsid mature protease, capsid protein, envelope glycoprotein B, single-stranded DNA-binding protein, DNA polymerase catalytic subunit, nuclear egress layer protein, DNA packaging protein UL32, DNA packaging protein UL33, nuclear egress membrane protein, large capsid protein, capsid triplex subunit 1, ribonucleotide reductase subunit 1, ribonucleotide reductase subunit 2, capsule host shutoff protein, DNA polymerase processing subunit, membrane protein UL45, coat protein VP13/14, trans-activating protein VP16, coatprotein VP22, envelope glycoprotein N, coat protein UL51, unwindase-primase primase subunit, envelope glycoprotein K, ICP27, nucleoproteinUL55, nucleoproteinUL56, transcription regulatory factorICP4, regulatory protein ICP22, envelope glycoprotein D, and membrane protein US8A.

Or, when the oncolytic virus is adenovirus, the essential gene is selected from the group consisting of early protein 1A, early protein 1B 19K, early protein 1B 55K, encapsidation protein Iva2, DNA polymerase, terminal protein precursor pTP, encapsidation protein 52K, capsid protein precursor pIIIa, pentomer matrix, core protein pVII, core protein precursor pX, core protein precursor pVI, hexonmer, proteinase, single-stranded DNA-binding protein, hexamer assembly protein 100K, protein 33K, encapsidation protein 22K, capsid protein precursor, protein U, fibrin, open reading frame 6/7 of regulatory protein E4, regulatory protein E4 34K, open reading frame 4 of regulatory protein E4, open reading frame 3 of regulatory protein E4, open reading frame 2 of regulatory protein E4, and open reading frame 1 of regulatory protein E4.

Or, when the oncolytic virus is vaccinia virus, the essential gene is selected from the group consisting of nucleotide reductase small-subunit, serine/threonine kinase, DNA-binding viral core protein, polymerase large-subunit, RNA polymerase subunit, DNA polymerase, sulfhydryl oxidase, hypothetical DNA-binding viral nucleoprotein, DNA-binding phosphoprotein, nucleoid cysteine proteinase, RNA helicase NPH-II, hypothetical metalloproteinase, transcription elongation factor, glutathione-type protein, RNA polymerase, hypothetical viral nucleoprotein, late transcription factor VLTF-1, DNA-binding viral nucleoprotein, viral capsid protein, polymerase small-subunit, RNA polymerase subunit rpo22 depending on DNA, RNA polymerase subunit rpo147 depending on DNA, serine/threonine protein phosphatase, IMV heparin-binding surface protein, DNA-dependent RNA polymerase, late transcription factor VLTF-4, DNA topoisomerase type I, mRNA capping enzyme large-subunit, viral core protein 107, viral core protein 108, uracil-DNA glycosylase, triphosphatase, 70kDa small subunit of early gene transcription factor VETF, RNA polymerase subunit rpo18 depending on DNA, nucleoside triphosphate hydrolase-I, mRNA capping enzyme small-subunit, rifampicin target site, late transcription factor VLTF-2, late transcription factor VLTF-3, disulfide bond forming pathway, precursor p4b of core protein 4b, core protein 39kDa, RNA polymerase subunit rpo19 depending on DNA, 82kDa large subunit of early gene transcription factor VETF, 32kDa small subunit of transcription factor VITF-3, IMV membrane protein 128, precursor P4a of core protein 4a, IMV membrane protein 131, phosphorylated IMV membrane protein, IMV membrane protein A17L, DNA unwindase, viral DNA polymerase processing factor, IMV membrane protein A21L, palmitoyl protein, 45kDa large subunit of intermediate gene transcription factor VITF-3, RNA polymerase subunit rpo132 depending on DNA, RNA polymerase rpo35 depending on DNA, IMV protein A30L, hypothetical ATP enzyme, serine/threonine kinase, EEV mature protein, palmitoylated EEV membrane glycoprotein, IMV surface protein A27L, EEV membrane phosphate glycoprotein, IEV and EEV membrane glycoproteins, EEV membrane glycoprotein, disulfide bond forming pathway protein, hypothetical viral nucleoprotein, IMV membrane protein I2L, poxvirus myristoyl protein, IMV membrane protein L1R, late 16kDa hypothetical membrane protein, hypothetical virus membrane protein H2R, IMV membrane protein A21L, chemokine-binding protein, epidermal growth factor-like protein, and IL-18 binding protein.

Or, when the oncolytic virus is coxsackie virus, the essential gene is selected from the group consisting of protein Vpg, core protein 2A, protein 2B, RNA unwindase 2C, protein 3A, proteinase 3C, reverse transcriptase 3D, coat protein Vp4, and protein Vp1.

Or, when the oncolytic virus is measles virus, the essential gene is selected from the group consisting of nucleoprotein N, phosphoprotein P, matrix protein M, transmembrane glycoprotein F, transmembrane glycoprotein H, and RNA-dependent RNA polymerase L.

Or, when the oncolytic virus is mumps virus, the essential gene is selected from the group consisting of nucleoprotein N, phosphoprotein P, fusion protein F, and RNA polymerase L.

Or, when the oncolytic virus is vesicular stomatitis virus, the essential gene is selected from the group consisting of glycoprotein G, nucleoprotein N, phosphoprotein P and RNA polymerase L.

Or, when the oncolytic virus is poliovirus, the essential gene is selected from the group consisting of capsid protein VP1, capsid protein VP2, capsid protein VP3, cysteine protease 2A, protein 2B, protein 2C, protein 3A, protein 3B, proteinase 3C, protein 3D, and RNA-directed RNA polymerase.

Or, when the oncolytic virus is influenza virus, the essential gene is selected from the group consisting of hemagglutinin, neuraminidase, nucleoprotein, membrane protein M1, membrane protein M2, polymerase PA, polymerase PB1-F2, and polymerase PB2.

In one or more embodiments of the present disclosure, a second regulatory element is inserted between the first and second codons of the open reading frame of one or more essential genes of the oncolytic virus.

In some embodiments of the present disclosure, the regulated viral essential genes can be more than one, and when several viral genes are regulated, the second regulatory element should be accordingly inserted between the first and second codons of the open reading frame of each essential gene.

In one or more embodiments of the present disclosure, the oncolytic virus is herpes simplex virus type 1, the essential gene is ICP27, the tumor-specific promoter is telomerase reverse transcriptase promoter, and the specific protease is the human rhinovirus 3C protease, the extracellular secretion signal peptide is interferon $\alpha$2 signal peptide, the amino acid sequence of the specific cleavage site is LEVLFQGP; the second regulatory element is located between the first and second codons of the open reading frame of the essential gene ICP27. The first regulatory element is located downstream the essential gene.

In one or more embodiments of the present disclosure,

the nucleotide sequence of the telomerase reverse transcriptase promoter is shown in SEQ ID NO: 4.

The amino acid sequence of human rhinovirus 3C protease is shown in SEQ ID NO: 5.

The nucleotide sequence of the open reading frame of human rhinovirus 3C protease is shown in SEQ ID NO: 6.

The amino acid sequence of the interferon $\alpha$2 signal peptide is shown in SEQ ID NO: 7; and the nucleotide sequence of the second nucleic acid sequence is shown in SEQ ID NO: 8.

The nucleotide sequence of the third nucleic acid sequence is shown in SEQ ID NO: 9.

In other aspect, the present disclosure provides a nucleic acid fragment for preparing the oncolytic virus, wherein the nucleic acid fragment consists of the 5' UTR of the essential gene, ATG, the second regulatory element, the remaining portion of the open reading frame of the essential gene without ATG, an exogenous Poly (A), the first regulatory element followed by the 3' UTR of the regulated essential gene. The 5' and 3' UTR sequences are used to facilitate the homologous recombination between the nucleic acid fragment-containing plasmid and viral genome for generation of the oncolytic virus.

In another aspect, the present disclosure provides an oncolytic virus containing the above-mentioned nucleic acid fragment.

In another aspect, the present disclosure provides a method for preparing oncolytic virus as described above, which comprises: infection of complementing cells with parental virus followed by transfection of the cells with plasmid DNA containing the nucleic acid fragment, and screening, confirmation and propagation of the oncolytic virus.

Herein, the genome of the parent virus lacks the regulated viral essential genes as compared to the genome of the wild-type virus.

Complementing cells constitutively expresses the regulated viral essential gene.

In another aspect, the present disclosure provides the use of the oncolytic virus as described above as a drug for killing tumor cells *in vitro.*

In another aspect, the present disclosure provides a drug for treating tumors, comprising the oncolytic virus as described above and pharmaceutically acceptable excipients.

Those skilled in the art can select an appropriate pharmaceutically acceptable excipient as required, and it is not limited in the present disclosure.

In one or more embodiments of the present disclosure, the drug also includes gene therapies or vaccines.

In another aspect, the present disclosure provides a method of treating a disease in animals including: Administration of the drugs provided in the present disclosure to animals,
wherein the disease is lung cancer, gastric cancer, liver cancer, rectal cancer, breast cancer, prostate cancer, brain tumor, colon cancer, cervical cancer, kidney cancer, ovarian cancer, head and neck cancer, melanoma, pancreatic cancer or esophageal cancer.

BRIEF DESCRIPTION OF DRAWINGS

Exemplary features of the present disclosure, its nature and various advantages will be apparent from the accompanying drawings and the following detailed description of various embodiments. Non-limiting and non-exhaustive embodiments are described with reference to the accompanying drawings. The sizes and relative positions of elements in the drawings are not necessarily drawn to scale. For example, the shapes of various elements are selected, enlarged, and positioned to improve drawing legibility. The particular shapes of the elements as drawn have been selected for ease of recognition in the drawings. One or more embodiments are described hereinafter with reference to the accompanying drawings in which

Figure 1: Schematic representation of the genome structure of the recombinant oncolytic virus provided in the present disclosure. A: generalized genome structure of oncolytic viruses provided in this disclosure, wherein the first regulatory element was located downstream the regulated essential gene, and the second regulatory element is located between the first and second codons of the open reading frame of the regulated essential gene. B: a specific embodiment of A, wherein the virus was HSV-1, the tumor-specific promoter was hTERT promoter, the enhancer was CMV enhancer, and the specific protease was HRV-3C protease, the regulated essential gene was ICP27, the extracellular secretion signal peptide was interferon $\alpha$2 signal peptide; the specific cleavage site was specifically recognized and cleaved by HRV-3C protease. The oncolytic virus constructed was named as oHSV-BJS.

Figure 2: Schematic showing of the parental plasmid pcDNA3.1-EGFP unitized for constructing a plasmid expressing HSV-1 ICP27. In the plasmid, EGFP is constitutively expressed under the control of CMV promoter and the plasmid contains the neomycin-resistant gene expression sequence

Figure 3: ICP27 expression from oncolytic virus oHSV-BJS in African green monkey kidney cells (Vero, normal cells). Vero cells were infected with 3 MOI (virus/cell) HSV-1 wild-type virus KOS or oncolytic virus oHSV-BJS. One day later, the cells were collected, RNAs and proteins were isolated. ICP27 mRNA was detected by reverse transcription combined with semi-quantitative PCR (A in the figure), and ICP27protein detected by Western blotting (B in the figure).

Figure 4: Expression of HRV-3C in four tumor cells. Four tumor cells were infected with 3 MOI oncolytic virus oHSV-BJS or KOS. 24 hours after infection, total RNA and protein were isolated. HRV-3C mRNA was analyzed by semi-quantitative PCR, and t ICP27 protein detected by Western blotting. A: HRV-3C mRNA; B: ICP27 protein.

Figure 5: Replication kinetics of wild-type virus KOS and oncolytic virus oHSV-BJS in tumor cells. Four tumor cells were infected with 0.1 MOI KOS or oHSV-BJS, respectively. At different day after infection, the cells and culture medium were collected. The virus titer for each viral stock was determined. A: cervical tumor Hela cells; B: cervical squamous carcinoma siHa cells; C: breast cancer SK-BR3 cells; D: breast cancer ME-180 cells.

Figure 6: Inhibition of tumor growth by oncolytic virus oHSV-BJS in animal tumor models. Tumor animal models were established. After the tumor grew to 50-80 $mm^3$, the oncolytic virus was injected into the tumor every 3 days for a total of 3 times. PBS (without oncolytic virus) was injected as a negative control. After the oncolytic virus was injected, the tumor size was tested twice a week. When the negative control animals needed to be euthanized, the experiment ended. A tumor growth curve was plotted based on the tumor size (A: lung cancer; B: gastric cancer; C: liver cancer; D: rectal cancer), and the relative inhibition rate (E) was calculated by comparing the tumor size in the test group at the end of the test with that observed in the negative control.

## DETAILED DESCRIPTION OF THE EMBODIMENTS

In order to demonstrate the features of the present disclosure, its nature and various advantages, exemplary embodiments were executed and are described in details below. All experiments were conducted using standard methods as described in literature. Reagents were purchased from commercial providers and used according to the instructions of the manufacturer.

As used herein, terms "base sequence" and "nucleotide sequence" can be used interchangeably, and generally refer to the composition and order of nucleotides arranged in DNA or RNA.

The term "primer" refers to a synthetic oligonucleotide, which is required for de novo nucleic acid synthesis.

After binding to a polynucleotide template, the primer is extended in 5' to 3' direction along the template catalyzed by DNA polymerase, hereby producing an extended duplex. Nucleotide addition during the extension is determined by the sequence of the template. A primer is typically 18-23 nucleotides in length. However, a primer length is determined by several factors including the nucleotide composition and the melting point of the primer, and the downstream application of the PCR product after amplified.

The term "promoter" generally refers to a DNA sequence that is located upstream the coding region of a gene, can be specifically identified and bound to by an RNA polymerase, and is required by transcription.

The term "enhancer" refers to a DNA sequence that increases transcription frequency of the gene interlocked therewith. The enhancer enhances the transcription by increasing the activity of a promoter. An enhancer may be located either at the 5' or the 3'end of a gene, and even may exist as an intron within a gene. An enhancer might significantly affect gene expression, which might increase the gene transcription by 10-200 folds, or even by thousand times.

Terms "subject", "individual", and "patient" can be used interchangeably herein, and refer to a vertebrate, preferably a mammal, most preferably human. The mammal comprises, but is not limited to, mouse, ape, human, domesticated animal, or farm-raised livestock.

The features and its nature of the present disclosure are described in detail below with reference to examples.

Example 1

The oncolytic virus provided in this example was generated by genetically engineering wild-type herpes simplex virus type 1 KOS. The genome of the oncolytic virus oHSV-BJS contains the following elements refer to Figure 1B).

(1) A first regulatory element is located downstream the essential gene ICP27 of the oncolytic virus; and the first regulatory element includes: tumor specific promoter, namely hTERT promoter, an enhancer, namely CMV enhancer, a nucleic acid sequence for encoding the specific protease, namely human rhinovirus 3C protease (HRV-3C protease) and BGH Poly(A).
Herein, the base sequence of hTERT promoter is shown in SEQ ID NO: 4;

the base sequence of the CMV enhancer is shown in SEQ ID NO: 10;
the nucleic acid fragment encoding HRV-3C protease is shown in SEQ ID NO: 6; and
the amino acid sequence of HRV-3C protease is shown in SEQ ID NO: 5.

(2) A second regulatory element is located between the first and second codons of the open reading frame of the essential gene ICP27 of the oncolytic virus; and the second regulatory element includes: the second nucleic acid sequence for encoding an extracellular secretion signal peptide, namely the interferon $\alpha$2 signal peptide, and the third nucleic acid sequence for encoding the specific cleavage site sequence.

Herein, the amino acid sequence of the interferon $\alpha$2 signal peptide is shown in SEQ ID NO: 7;

the nucleotide sequence of the second nucleic acid sequence for encoding the interferon $\alpha$2 signal peptide is shown in SEQ ID NO: 8;
the amino acid sequence of the specific cleavage site is LEVLFQGP; and
the nucleotide sequence of the third nucleic acid sequence for encoding the specific cleavage site, is TTAGAAGTTCTTTTTCAAGGTCCT.

When the oncolytic virus infects normal cells, the HRV-3C protease is not expressed. Therefore, the specific cleavage site will be not cleaved, and the interferon $\alpha$2 extracellular secretion signal peptide will direct the secretion of the ICP27 fusion protein to the outside of the cells, resulting in no viral replication. Therefore, the virus is safe to normal cells. When the oncolytic virus infects tumor cells, the HRV-3C protease is specifically expressed under the control of hTERT promoter, and the specific cleavage site will be recognized and cleaved by the expressed HRV-3C protease, and the ICP27 protein can be partitioned and localized normally in the tumor cells. Therefore, the oncolytic virus replicates normally and kill target tumor cells.

The preparation of the above-mentioned oncolytic virus oHSV-BJS provided in this example was as follows.

(1) Preparation of complementing cells expressing HSV-1 ICP27

(a) plasmid construction: Using the DNA of wild-type herpes simplex virus type 1 KOS as a template, the encoding region of ICP27 was amplified by PCR, and inserted into HindIII and XbaI sites of the plasmid pcDNA3.1-EGFP (Figure 2) to replace EGFP. The recombinant plasmid was named as ICP27 expression plasmid. The expression of ICP27 from the plasmid is driven under the control of CMV promoter.

(b) G418 dose determination for selection: Vero cells were treated with G418 of different concentrations, the culture medium containing G418 was replaced every three days with media containing G418 of different concentrations, and cell death was monitored every day. The minimal concentration of G418 required for all the cells to die after 6 days of G418 treatment was determined. Such a concentration of G418 (500 μg/ml) was utilized for complementing cell establishment.

(c) Cell line establishment: $3.5\times10^5$ Vero cells were seeded into each well of a 6-well cell culture plate and cultured overnight in an antibiotic-free culture medium, and 4 μg of the ICP27 expression plasmid DNA obtained from step (a) were transfected into cells in each well using Lipofectamine 2000. After 24 hours of culture, cells in each well were harvested, and diluted by 20, 40, or 60-fold. Cells were cultured in the culture medium containing 500 μg/ml G418, and the medium replaced with fresh medium containing G418 every 3 days. After 6-7 times of medium change, the clones were collected and propagated step by step from the 24-well plate to T150 tissue culture flasks. Subsequently, the protein was isolated, and the expression of ICP27 detected by western blotting. The Cells with the highest level of ICP27 expression were selected as the complementing cells to support the growth and replication of replication-defective viruses in which ICP27 are not expressed; the cells were named as $C_{ICP27}$

The complementing $C_{ICP27}$ cells have been preserved at China Center for Type Culture Collection (CCTCC), Wuhan University, Luojiashan, Wuchang, Wuhan City on April 24, 2019 with a preservation number of CCTCC NO. C201974.

(2) Preparation of the parental virus HSV-EGFP

The wild-type type 1 herpes simplex virus KOS was used as the starting material. The recombinant parental virus HSV-EGFP was obtained by homologous recombination between plasmid and KOS genome. In HSV-EGFP, HSV-ICP27 was replaced by EGFP. HSV-EGFP served as the parental virus for generating the oncolytic viruses provided in this disclosure. The detailed manipulations were as the follows.

(a) the first nucleic acid fragment was synthesized and its nucleotide sequence is shown in SEQ ID NO. 1:
The fragment includes the following elements: ICP27 5' sequence, CMV promoter, Kozak sequence, EGFP encoding frame, BGH Poly(A) and ICP27 3' sequence.
Sequence seen in SEQ ID NO. 1:

site 1-6: irrelevant sequence, increasing the end length to facilitate enzyme digestion;
site 7-12: Xho1 site, C/TCGAG;
site 13-575: ICP27 5' UTR without ICP27 endogenous promoter;
site 576-1163: CMV promoter;
site 1164-1174: interval sequence;
site 1175-1180: Kozak sequence, increasing protein expression;
site 1181-1900: EGFP encoding frame;
site 1901-2145: BGH Poly(A);
site 2146-2667: ICP27 3' UTR;
site 2668-2673: Hindlll site, A/AGCTT; and
site 2674-2679: irrelevant sequence, increasing the end length to facilitate enzyme digestion.

(b) the first fragment was cleaved and ligated to the Hindlll and Xho1 sites of the pcDNA3.1-EGFP plasmid, and the resulting recombinant plasmid was named as EGFP expression plasmid.

(c) $3.5\times10^5$ complementing $C_{ICP27}$ cells were seeded into each well of 6-well cell culture plate and cultured overnight in an antibiotic-free culture medium.

(d) the cells were infected with 0.1, 0.5, 1, 3 MOI wild-type virus KOS (virus/cell), respectively. One hour later, the above-mentioned EGFP expression plasmid (4 μg DNA/well) was transfected into the cells using Lipofectamine 2000. After 4 hours of incubation, the transfection mixture was replaced with complete medium. When all the cells became spherical, the cells and culture medium were collected. The mixture was centrifuged after three cycles of freeze-thawing and the supernatant collected, The virus stocks were diluted, and infected complementing $C_{ICP27}$ cells. Viruses were separated using plaque separation method. 4-5 days later, the virus plaque with the strongest green fluorescence was selected and picked under a fluorescence microscope. The obtained virus plaque was subjected to 2 or 3 rounds of screening to obtain pure virus plaques. The virus was propagated. The recombinant virus with ICP27

replaced by EGFP was named as HSV-EGFP. HSV-EGFP served as the parental virus for generation of oncolytic virus oHSV-BJS provided in this disclosure.

(3) Construction of recombinant plasmid containing the first expression regulatory element and the second regulatory element

(a) the TA cloning plasmid was modified such that the multiple cloning site only contains the XhoI site, and the resulting plasmid named as TA-XhoI plasmid.
(b) the second nucleic acid fragment was synthesized with sequence shown in SEQ ID NO:2. The fragment includes the following elements:
ICP27 5' UTR including the endogenous promoter, ATG, the second nucleic acid sequence for encoding interferon $\alpha$2 extracellular secretion signal peptide, the third nucleic acid sequence for encoding the specific cleavage site recognized and cleaved by HRV-3C protease, ICP27 open reading frame sequence without ATP, SV40 Poly(A), ICP27 3' UTR, wherein there was a HindIII site between SV40 Poly(A) and ICP27 3' UTR, which is used to insert the first regulatory element. Detailed information of each element in the fragment is as follows:

site 1-6: Xho1 site;
site 7-677: ICP27 5' UTR containing endogenous ICP27 promoter;
site 678-746: the second nucleic acid sequence for encoding the interferon $\alpha$2 signal peptide;
site 747-770: the third nucleic acid sequence for encoding the specific cleavage site of HRV-3C protease;
site 771-2306: ICP27 open reading frame (without start codon ATG);
site 2307-2753: SV40 Poly(A);
site 2754-2759: HindIII site;
site 2760-3279: ICP27 3' UTR; and
site 3280-3285: Xho1 site.

The second nucleic acid fragment was cleaved by Xho 1, ligated into the Xho1 site of plasmid TA-XhoI, and the resulting plasmid was named as pTA-XhoI-S-ICP27.
(c) the third nucleic acid fragment was synthesized with sequence shown in SEQ ID NO:3. It contains the following elements: hTERT promoter, CMV enhancer, Kozak sequence, the nucleic acid sequence for encoding the HRV-3C protease and SV40 Poly(A). Detailed information of each element in the fragment is as follows:

site 1-6: HindIII site;
site 7-432: hTERT promoter;
site 433-527: CMV enhancer;
site 528-539: Kozak sequence;
site540-1088: nucleic acid sequence for encoding HRV-3C protease;
site 1089-1544: SV40 Poly(A); and
site 1545-1550: HindIII site.

The third nucleic acid fragment was cleaved by HindIII and ligated into pTA-XhoI-S-ICP27, and the obtained plasmid was named as pTA-XhoI-S-ICP27-3C plasmid.

(4) Construction of recombinant oncolytic herpes viruses

(a) $3.5\times10^5$ complementing $C_{ICP27}$ cells were seeded into each well of a 6-well cell culture plate and cultured overnight in an antibiotic-free culture medium.
(b) the complementing $C_{ICP27}$ cells were infected with 0.1, 0.5, 1, 3 MOI (virus/cell) of the parent virus HSV-EGFP, respectively. After 1 hour incubation, pTA-XhoI-S-ICP27-3C DNA (4$\mu$g DNA/well) was transfected into the cells using Lipofectamine 2000. After 4 hours of incubation, the transfection mixture was replaced with complete medium. When all the cells became spherical, the cells and culture medium were collected. The mixture was centrifuged after three cycles of freeze-thawing, the supernatant collected. Virus stocks were diluted, and infected the complementing $C_{ICP27}$ cells. The viruses were isolated using plaque separation method. 4-5 days after infection, virus plaques without green fluorescence under a fluorescence microscope were picked. The obtained virus plaques were subjected to 2 or 3 rounds of screening to obtain pure virus plaques, the virus was propagated and expanded, the infected cell DNA was isolated, and the recombinant oncolytic viruses were confirmed by PCR amplification and sequencing, and the oncolytic virus was named as oHSV-BJS.

The recombinant oncolytic virus oHSV-BJS was preserved in the Chinese Center of Type Culture Collection (CCTCC), a Wuhan University, Luojiashan, Wuchang, Wuhan, China on April 24, 2019. The preservation number is CCTCC NO: V201920.

**Experimental example 1**

Detection of the expression of ICP27 from the recombinant oncolytic virus oHSV-BJS in Vero cells

Method: Vero was infected with 3 MOI wild-type KOS and oncolytic virus oHSV-BJS, respectively. One day after infection, cells were collected, RNAs and proteins were isolated. ICP27 mRNA was detected by reverse transcription combined with semi-quantitative PCR, and tICP27 protein detected by Western blotting. For mRNA and protein detection, β-actin was used as the loading control.

Results: oHSV-BJSthere was no significant difference in ICP27 mRNA level in oHSV-BJS-infected vero cells compared to KOS-infected vero cells ( Figure 3A) .However, ICP27 protein in Vero cells infected with oHSV-BJS was significantly lower than that observed in Vero cells infected with KOS (Figure 3B)). The results suggest ICP27 protein once produced is secreted to the outside of the cells.

**Experimental example 2**

Detection of the expression of HRV-3C protease and ICP27 in tumor cells

Method: Four tumor cells were infected with 3 MOI oncolytic virus oHSV-BJS or KOS. 24 hours after infection, total RNA and protein were isolated. HRV-3C mRNA was analyzed by semi-quantitative PCR, and t ICP27 protein detected by Western blotting. For mRNA and protein detection, β-actin was used as the loading control.

Results: HRV-3C mRNA was expressed to a detectable level in all the four tumor cells infected with oHSV-BJS (Figure 4 A). ICP27 protein was accumulated to a detectable level in all the four tumor cells infected with the oncolytic virus oHSV-BJS. Moreover, there was no significant difference in ICP27 protein between oncolytic virus oHSV-BJS and KOS infected cells for a given cell type (Figure 4B).

The results demonstrate that HRV-3C was specifically expressed in tumor cells and cleaved ICP27 fusion protein.

**Experimental** example 3

Replication kinetics of recombinant oncolytic virus oHSV-BJS

Method: tumor cells were infected with 0.1 MOI KOS or oHSV-BJS, respectively. At different day after infection, the cells and culture medium were collected, and the viruses remaining in the cells were released into the culture medium through three cycles of -80/37°C freeze-thawing. The complementing $C_{ICP27}$ cells were then infected with the virus, and the virus titer (plaque forming unit/ml, PFU/ml) was determined by plaque assay.

Results: with the replication kinetics of oHSV-BJS are quite different from one cell to another cell type. But for a given cell type, there is no significant difference in viral replication in bother oHSV-BJS-infected and KOS-infected cells (Figure 5A-D). the results indicate that genetic modification used for generating the oncolytic virus provided in this disclosure does not significantly alter the replication capacity in tumor cells.

**Experimental** example 4

The ability of recombinant oncolytic virus oHSV-BJS to kill tumor cells

Methods: tumor cells were infected by MOI 0.25 or 0.5 KOS or oHSV-BJS respectively. Cell viability was assayed at different day after infection

0.25 or 0.5 MOI oHSV-BJS shows a varied ability to kill different tumor cells. But for a given cell type, the efficiency of oHSV-BJS in killing cells was basically the same as that observed for KOS (Table 1-4). the results indicate that oncolytic virus oHSV-BJS retains the ability of wild-type KOS virus to kill tumor cells.

Table 1. Viability of (%) of Hela cells infected with oHSV-BJS at different day after infection

| virus (MOI 0.25) | oHSV-BJS | KOS |
|---|---|---|
| the first day | 58±3.0 | 46±2.0 |
| the second day | 38±2.0 | 29±1.4 |
| the third day | 17±0.8 | 11±0.6 |
| the fourth day | 4±0.3 | 0 |
| virus (MOI 0.5) | oHSV-BJS | KOS |
| the first day | 52±2.7 | 39±1.8 |
| the second day | 28±1.8 | 22±1.1 |
| the third day | 9±0.1 | 5±0.3 |
| the fourth day | 0 | 0 |

Table 2. Viability (%) of siHA cells infected with oHSV-BJS at different day after infection

| virus (MOI 0.25) | oHSV-BJS | KOS |
|---|---|---|
| the first day | 22±1.4 | 16±1.0 |
| the second day | 6±0.4 | 0 |
| the third day | 0 | 0 |
| virus (MOI 0.5) | oHSV-BJS | KOS |
| the first day | 8±0.4 | 4±0.3 |
| the second day | 1±0.1 | 0 |
| the third day | 0 | 0 |

Table 3. Viability (%) of SK-BR3 cells infected with oHSV-BJS at different day after infection

| virus (MOI 0.25) | oHSV-BJS | KOS |
|---|---|---|
| the first day | 21±1.3 | 6±0.4 |
| the second day | 0 | 0 |
| the third day | 0 | 0 |
| virus (MOI 0.5) | oHSV-BJS | KOS |
| the first day | 3±0.1 | 5±0.3 |
| the second day | 0 | 0 |
| the third day | 0 | 0 |

Table 4. viability (%) of ME-180 cells infected with oHSV-BJS at different day after infection

| virus (MOI 0.25) | oHSV-BJS | KOS |
|---|---|---|
| the first day | 12±0.8 | 10±0.5 |
| the second day | 4±0.3 | 0 |
| the third day | 0 | 0 |
| virus (MOI 0.5) | oHSV-BJS | KOS |

(continued)

| virus (MOI 0.25) | oHSV-BJS | KOS |
|---|---|---|
| the first day | 5±0.5 | 5±0.4 |
| the second day | 1±0.10 | 0 |
| the third day | 0 | 0 |

**Experimental** example 5

Effect of recombinant oncolytic virus oHSV-BJS on the viability of normal cells
Methods: Vero cells or primary human corneal epidermal cells were infected with oncolytic virus oHSV-BJS (2 MOI) or wild virus KOS (0.5 MOI) respectively. Viability of the cells infected with oHSV-BJS were assayed 3 days after infection while the viability of the cells infected with wild virus KOS were measured 2 days after infection. All Vero cells or primary human corneal epidermal cells died 2 days after KOS infection. But the viability of the cells infected with the oncolytic virus oHSV-BJS was basically the same as that of observed for mock treatment (not treated) (Table 5). The results indicate that oncolytic virus oHSV-BJS is safe to normal cells.

Table 5. viability (%) of normal cells 3 days after oHSV-BJS infection or 2 days after KOS infection

| virus | oHSV-BJS | KOS | untreated |
|---|---|---|---|
| Vero cells | 95±2 | 0 | 98±2 |
| corneal epidermal cells | 97±2 | 0 | 97±1 |

**Experimental example 6**

Detection of the inhibition of tumor growth by oncolytic virus oHSV-BJS *in vivo*

Method: To establish animal tumor models of lung cancer (A549 cells), gastric cancer (NCI-N87 cells), liver cancer (SK-HEP-1 cells) and rectal cancer (HCT-8), tumor cells were subcutaneously inoculated into mice. When the tumors grew to 40-120mm$^3$, oncolytic virus oHSV-BJS was injected into the tumors once every 3 days for a total of 3 times of injection, $1.2\times10^7$ infection units (suspended in 40μl PBS) were injected for each time at multiple sites. Mice injected with PBS (without oncolytic virus) were used as negative control, with 8 animals in each experiment group. After the injection of oncolytic virus, the tumor sizes were measured twice a week (the relative tumor size was defined as 1 at the first injection) for a total of 17-32 days (depending on the time when the animal in the negative control group needed to be euthanized). The tumor growth curve was plotted according to the tumor size. At the end of the experiment, the tumor size was measured and compared with that in the negative control, and the inhibition rate was calculated.

inhibition rate (%) = (tumor volume of negative control group -tumor volume of test group)/tumor volume in negative control group x 100%.

oHSV-BJS slowed down the growth of lung, gastric, liver and rectal tumors (Figure 6A-D). The inhibition of oHSV-BJS on lung, gastric, liver and rectal tumor were 45%, 37%, 26% and 49%, respectively (Figure 6E). The results showed that oHSV-BJS can inhibit the proliferation of various tumors.
The foregoing descriptions are only typical examples of the present disclosure, and are not intended to limit the scope of the present disclosure. Any modification, equivalent replacement, improvement, etc. made within the concept and principle of the present disclosure shall be included in the protection scope of the present disclosure.

**Industrial applicability**

The oncolytic virus provided by the present disclosure can be produced on industrial scale. The oncolytic virus has not deleted any genes (either essential or non-essential genes) from its original genome. It can replicate with high capability and kill tumor cells, which can be used to treat cancer and it is safe to non-cancer cells.

## Claims

1. An oncolytic virus, **characterized in that** a first regulatory element is inserted into the viral genome thereof, wherein the first regulatory element comprises a tumor-I specific promoter and a first nucleic acid sequence that is driven by the tumor -specific promoter to express a specific protease in target cancer cells;

   a second regulatory element is further inserted into the genome of the oncolytic virus, wherein the second regulatory element comprises a second nucleic acid sequence for encoding an extracellular secretion signal peptide and a third nucleic acid sequence for encoding a specific cleavage site; and the specific cleavage site is recognized and cleaved by the specific protease; and
   the second regulatory element is located between the first and second codons of the regulated essential gene of the oncolytic virus.

2. The oncolytic virus of claim 1, wherein the specific protease is selected from the group consisting of human rhinovirus 3C protease, thrombin, factor Xa protease, tobacco etch virus protease or recombinant PreScission protease, wherein

   preferably, when the specific protease is human rhinovirus 3C protease, anamino acid sequence of the specific cleavage site is LEVLFQGP;
   when the specific protease is thrombin, the amino acid sequence of the specific cleavage site is LVPRGS;
   when the specific protease is factor Xa protease, the amino acid sequence of the specific cleavage site is IE/DGR;
   when the specific protease is tobacco etch virus protease, the amino acid sequence of the specific cleavage site is ENLYFQG; and
   when the specific protease is a recombinant PreScission protease, the amino acid sequence of the specific cleavage site is LEVLFQGP.

3. The oncolytic virus of claim 1 or 2, wherein the extracellular secretion signal peptide is selected from the group including interferon $\alpha 2$, interleukin 2, human serum albumin, human immunoglobulin heavy chain, and luciferase extracellular secretion signal peptides.

4. The oncolytic virus of any one of claims 1 to 3, wherein the second regulatory element is located between the first and second codons of the open reading frame of the regulated essential gene of the oncolytic virus.

5. The oncolytic virus of any one of claims 1 to 4, wherein the first regulatory element further comprises an enhancer, and the enhancer is located between the tumor-specific promoter and the encoding sequence of the specific protease, the enhancer is used to enhance expression of the specific protease; and
preferably, the enhancer is either CMV enhancer or SV40 enhancer.

6. The oncolytic virus of any one of claims 1- 5, wherein the target cancer cells are lung cancer cells, liver cancer cells, breast cancer cells, gastric cancer cells, prostate cancer cells, brain tumor cells, human colon cancer cells, cervical cancer cells, kidney cancer cells, ovarian cancer cells, head and neck cancer cells, melanoma cells, pancreatic cancer cells, or esophageal cancer cells.

7. The oncolytic virus of any one of claims 1-6, wherein the tumor- specific promoter is selected from the group consisting of telomerase reverse transcriptase promoter, human epidermal growth factor receptor-2 promoter, E2F1 promoter, osteocalcin promoter, carcinoembryonic antigen promoter, survivin promoter and ceruloplasmin promoter.

8. The oncolytic virus of any one of claims 1-7, wherein the oncolytic virus is selected from the group consisting of herpes simplex virus, adenovirus, vaccinia virus, newcastle disease virus, poliovirus, coxsackie virus, measles virus, mumps virus, vesicular stomatitis virus and influenza virus,

preferably, when the oncolytic virus is herpes simplex virus, the essential gene is selected from the group consisting of envelope glycoprotein L, uracil DNA glycosylase, capsid protein, helicase proenzyme subunit, DNA replication initiation binding unwindase, derived protein of myristic acid, deoxyribonuclease, coat serine/threonine protein kinase, DNA packaging terminase subunit 1, coat protein UL16, DNA packaging protein UL17, capsid triplex subunit 2, major capsid protein, envelope protein UL20, nucleoprotein UL24, DNA packaging protein UL25, capsid mature protease, capsid protein, envelope glycoprotein B, single-stranded DNA-binding protein, DNA polymerase catalytic subunit, nuclear egress layer protein, DNA packaging protein UL32, DNA packaging protein UL33, nuclear egress membrane protein, large capsid protein, capsid triplex subunit 1, ribonucleotide reductase subunit 1, ribonucleotide reductase subunit 2, capsule host shutoff protein, DNA polymerase processing subunit, membrane protein UL45, coat protein VP13/14, trans-activating protein VP16, coatprotein VP22, envelope glycoprotein N, coat protein UL51, unwindase-primase primase subunit, envelope glycoprotein K, ICP27, nucleoproteinUL55, nucleoproteinUL56, transcription regulatory factorICP4, regulatory protein ICP22, envelope glycoprotein D, and membrane protein US8A;
preferably, when the oncolytic virus is adenovirus, the essential gene is selected from the group consisting of early protein 1A, early protein 1B 19K, early protein 1B 55K, encapsidation protein Iva2, DNA polymerase, terminal protein precursor pTP, encapsidation protein 52K, capsid protein precursor pIIIa, pentomer matrix, core protein pVII, core protein precursor pX, core protein precursor pVI, hexonmer, proteinase, single-stranded DNA-binding protein, hexamer assembly protein 100K, protein 33K, encapsidation protein 22K, capsid protein precursor, protein U, fibrin, open reading frame 6/7 of regulatory protein E4, regulatory protein E4 34K, open reading frame 4 of regulatory protein E4, open reading frame 3 of regulatory protein E4, open reading frame 2 of regulatory protein E4, and open reading frame 1 of regulatory protein E4;
preferably, when the oncolytic virus is vaccinia virus, the essential gene is selected from the group consisting of nucleotide reductase small-subunit, serine/threonine kinase, DNA-binding viral core protein, polymerase large-subunit, RNA polymerase subunit, DNA polymerase, sulfhydryl oxidase, hypothetical DNA-binding viral nucleoprotein, DNA-binding phosphoprotein, nucleoid cysteine proteinase, RNA helicase NPH-II, hypothetical metalloproteinase, transcription elongation factor, glutathione-type protein, RNA polymerase, hypothetical viral nucleoprotein, late transcription factor VLTF-1, DNA-binding viral nucleoprotein, viral capsid protein, polymerase small-subunit, RNA polymerase subunit rpo22 depending on DNA, RNA polymerase subunit rpo147 depending on DNA, serine/threonine protein phosphatase, IMV heparin-binding surface protein, DNA-dependent RNA polymerase, late transcription factor VLTF-4, DNA topoisomerase type I, mRNA capping enzyme large-subunit, viral core protein 107, viral core protein 108, uracil-DNA glycosylase, triphosphatase, 70kDa small subunit of early gene transcription factor VETF, RNA polymerase subunit rpo18 depending on DNA, nucleoside triphosphate hydrolase-I, mRNA capping enzyme small-subunit, rifampicin target site, late transcription factor VLTF-2, late transcription factor VLTF-3, disulfide bond forming pathway, precursor p4b of core protein 4b, core protein 39kDa, RNA polymerase subunit rpo19 depending on DNA, 82kDa large subunit of early gene transcription factor VETF, 32kDa small subunit of transcription factor VITF-3, IMV membrane protein 128, precursor P4a of core protein 4a, IMV membrane protein 131, phosphorylated IMV membrane protein, IMV membrane protein A17L, DNA unwindase, viral DNA polymerase processing factor, IMV membrane protein A21L, palmitoyl protein, 45kDa large subunit of intermediate gene transcription factor VITF-3, RNA polymerase subunit rpo132 depending on DNA, RNA polymerase rpo35 depending on DNA, IMV protein A30L, hypothetical ATP enzyme, serine/threonine kinase, EEV mature protein, palmitoylated EEV membrane glycoprotein, IMV surface protein A27L, EEV membrane phosphate glycoprotein, IEV and EEV membrane glycoproteins, EEV membrane glycoprotein, disulfide bond forming pathway protein, hypothetical viral nucleoprotein, IMV membrane protein I2L, poxvirus myristoyl protein, IMV membrane protein L1R, late 16kDa hypothetical membrane protein, hypothetical virus membrane protein H2R, IMV membrane protein A21L, chemokine-binding protein, epidermal growth factor-like protein, and IL-18 binding protein;
preferably, when the oncolytic virus is coxsackie virus, the essential gene is selected from the group consisting of protein Vpg, core protein 2A, protein 2B, RNA unwindase 2C, protein 3A, proteinase 3C, reverse transcriptase 3D, coat protein Vp4, and protein Vp1;
preferably, when the oncolytic virus is measles virus, the essential gene is selected from the group consisting of nucleoprotein N, phosphoprotein P, matrix protein M, transmembrane glycoprotein F, transmembrane glycoprotein H, and RNA-dependent RNA polymerase L;
preferably, when the oncolytic virus is mumps virus, the essential gene is selected from the group consisting of nucleoprotein N, phosphoprotein P, fusion protein F, and RNA polymerase L;
preferably, when the oncolytic virus is vesicular stomatitis virus, the essential gene is selected from the group consisting of glycoprotein G, nucleoprotein N, phosphoprotein P and RNA polymerase L;
preferably, when the oncolytic virus is poliovirus, the essential gene is selected from the group consisting of capsid protein VP1, capsid protein VP2, capsid protein VP3, cysteine protease 2A, protein 2B, protein 2C, protein 3A, protein 3B, proteinase 3C, protein 3D, and RNA-directed RNA polymerase; and
preferably, when the oncolytic virus is influenza virus, the essential gene is selected from the group consisting of hemagglutinin, neuraminidase, nucleoprotein, membrane protein M1, membrane protein M2, polymerase PA, polymerase PB1-F2, and polymerase PB2.

**9.** The oncolytic virus of any one of claims 1-8, wherein the second regulatory element is inserted between the first and second codons of one or more essential genes of the oncolytic virus.

**10.** The oncolytic virus of any one of claims 1-8, wherein the oncolytic virus is herpes simplex virus type 1, the essential gene is ICP27, the tumor-specific promoter is telomerase reverse transcriptase promoter, the specific protease is human rhinovirus 3C protease, the extracellular secretion signal peptide is interferon $\alpha 2$ signal peptide, the amino acid sequence of the specific cleavage site is LEVLFQGP; the second regulatory element is located between the first and second codons of the open reading frame of the essential gene ICP27; and the first regulatory element is located downstream the essential gene.

**11.** The oncolytic virus of claim 10, wherein

The nucleotide sequence of the telomerase reverse transcriptase promoter is shown in SEQ ID NO: 4;
The amino acid sequence of the human rhinovirus 3C protease is shown in SEQ ID NO: 5;
The nucleotide sequence of the open reading frame of the human rhinovirus 3C protease is shown in SEQ ID NO: 6;
The amino acid sequence of the interferon $\alpha 2$ signal peptide is shown in SEQ ID NO: 7; The nucleotide sequence of the second nucleic acid sequence is shown in SEQ ID NO: 8; and
The nucleotide sequence of the third nucleic acid sequence is shown in SEQ ID NO: 9.

**12.** The oncolytic virus of claim 1, wherein the insertion of the first regulatory element is located between two genes of the oncolytic virus.

**13.** The oncolytic virus of claim 12, wherein the two genes can be two essential genes, or one of them is an essential gene and the other is a non-essential gene.

**14.** A nucleic acid fragment for preparing the oncolytic virus of any one of claims 1-13, **characterized in that** the exogenous sequence is inserted into the target site of the oncolytic virus, and the exogenous sequence comprises the first regulatory element and the second regulatory element, the first regulatory element is located between two genes of the oncolytic virus, and the second regulatory element is located between the first and second codons of the essential gene of the oncolytic virus.

**15.** The nucleic acid fragment of claim 14, wherein the fragment contains 5' and 3' arms with sequences homologous to the sequences upstream and downstream the encoding region of the regulated gene, which serves as the sequence basis for inserting the fragment into the viral genome.

**16.** A vector containing the nucleic acid fragment of claim 14 or 15.

**17.** A method for preparing the oncolytic virus of any one of claims 1-13, **characterized in that** the method comprises: infection of complementing cells with parental virus followed by transfection of the cells with plasmid DNA containing the nucleic acid fragment, screening, confirmation and propagation of the oncolytic virus.
The genome of the parent virus lacks the regulated essential gene; and
the complementing cells constitutively expresses the regulated essential gene.

**18.** Use of the oncolytic virus of any one of claims 1-13 to prepare a drug for treating cancer.

**19.** A drug for treating cancer, **characterized in that** the drug comprises the oncolytic virus of any one of claims 1-13 and pharmaceutically acceptable excipients.

**20.** The drug according to claim 19, wherein the drug further comprises gene therapy drug or vaccine.

**21.** A method for treating a disease in a subject, **characterized by** comprising

administration of the drug of claim 19 or 20 to the subject,
wherein the disease is lung cancer, gastric cancer, liver cancer, rectal cancer, breast cancer, prostate cancer, brain tumor, colon cancer, cervical cancer, kidney cancer, ovarian cancer, head and neck cancer, melanoma, pancreatic cancer or esophageal cancer.

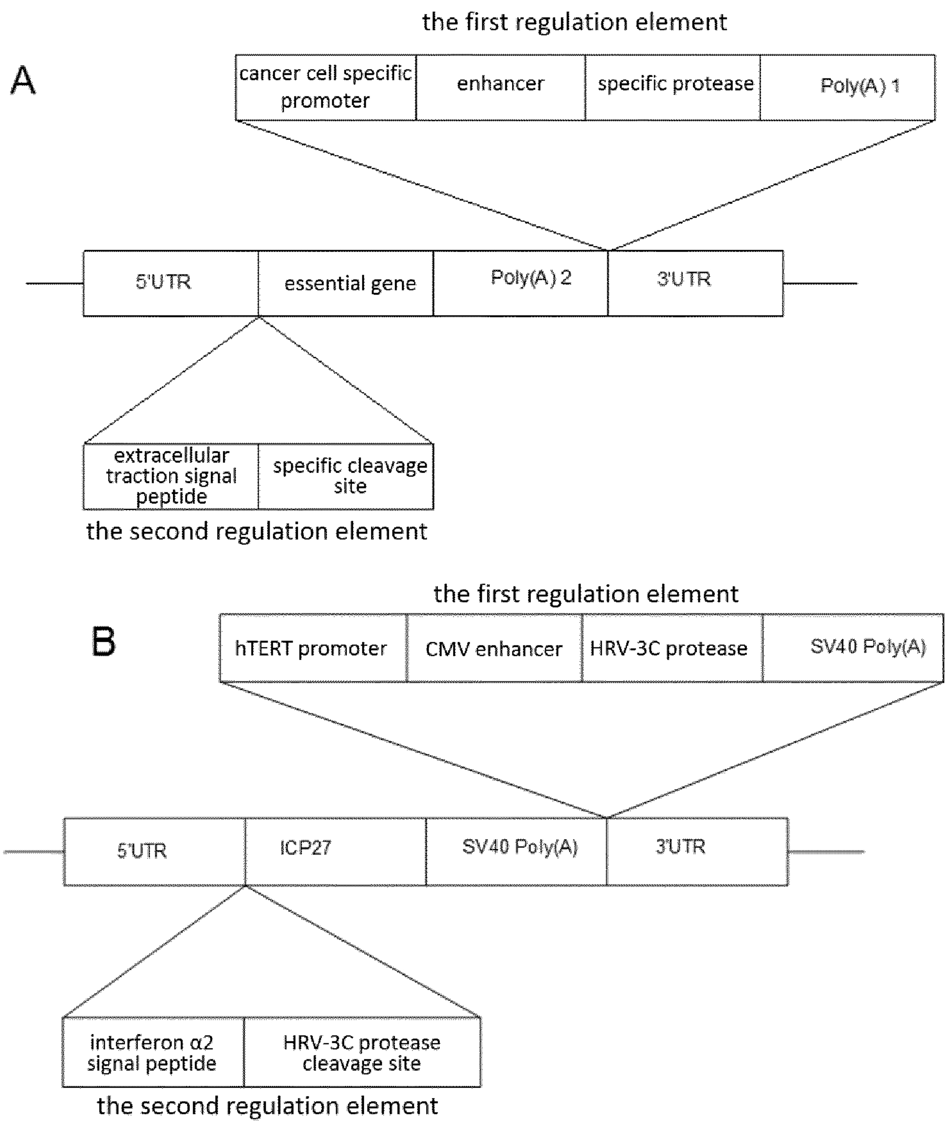
the first regulation element
A
cancer cell specific promoter
enhancer
specific protease
Poly(A) 1
5'UTR
essential gene
Poly(A) 2
3'UTR
extracellular traction signal peptide
specific cleavage site
the second regulation element
the first regulation element
B
hTERT promoter
CMV enhancer
HRV-3C protease
SV40 Poly(A)
5'UTR
ICP27
SV40 Poly(A)
3'UTR
interferon α2 signal peptide
HRV-3C protease cleavage site
the second regulation element

Figure. 1

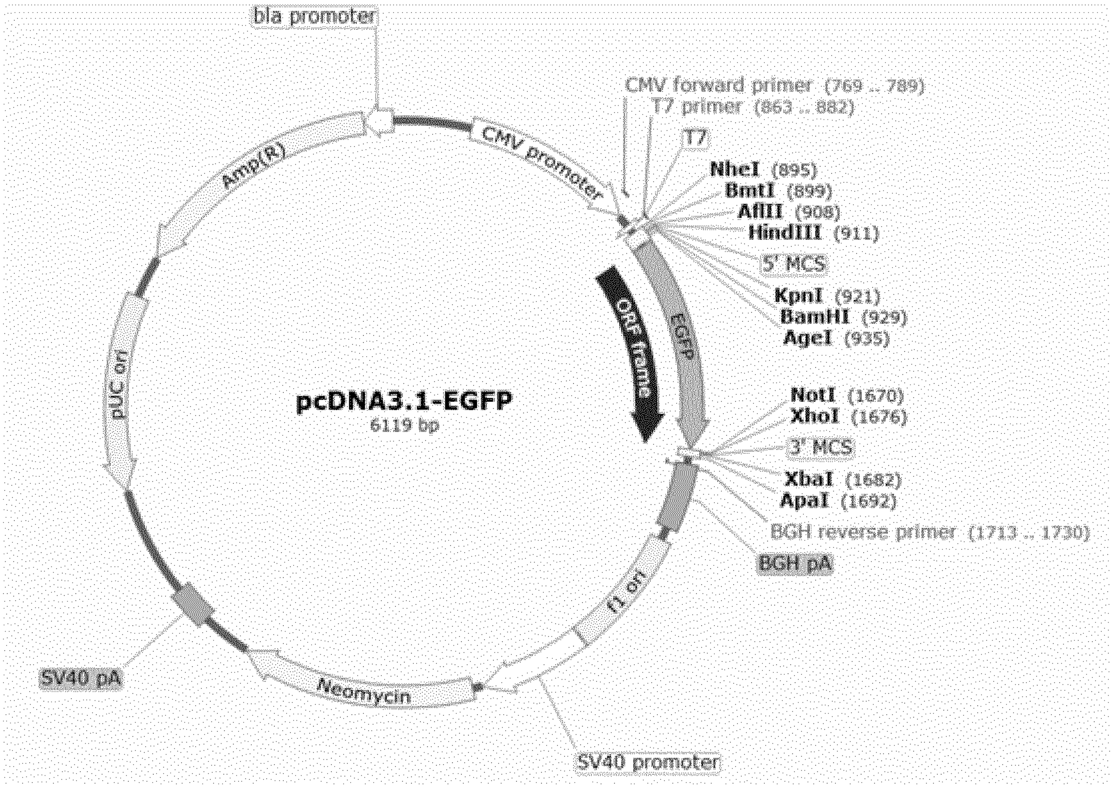


Figure. 2

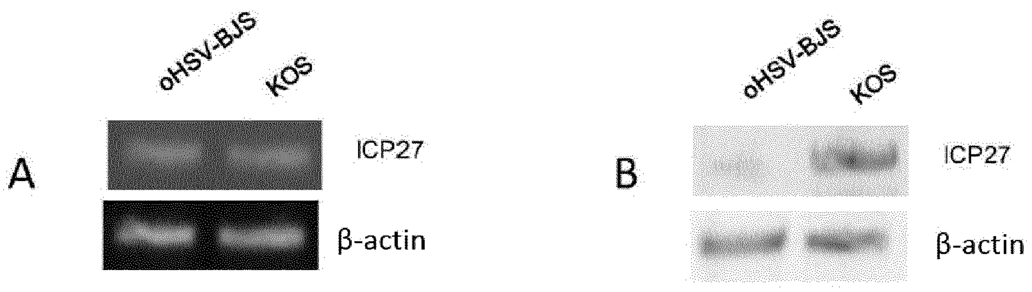


Figure. 3

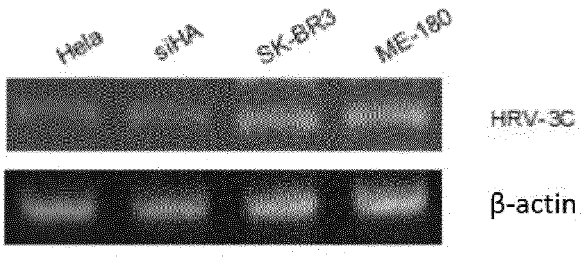
Hela
siHA
SK-BR3
ME-180
HRV-3C
β-actin

A

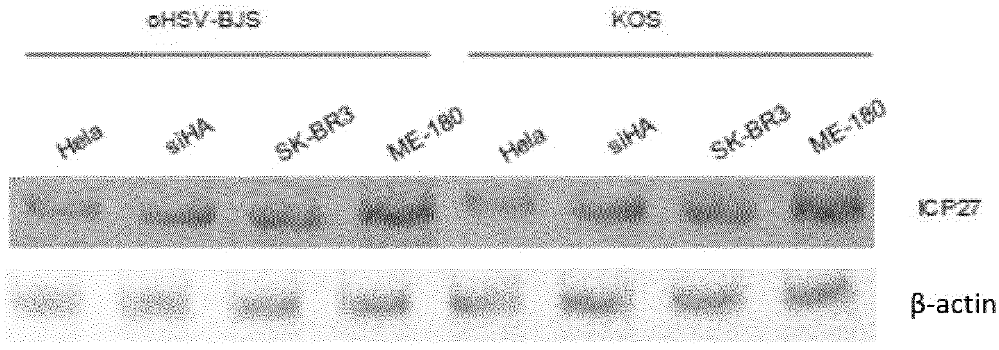
oHSV-BJS
KOS
Hela
siHA
SK-BR3
ME-180
Hela
siHA
SK-BR3
ME-180
ICP27
β-actin

B

Figure. 4

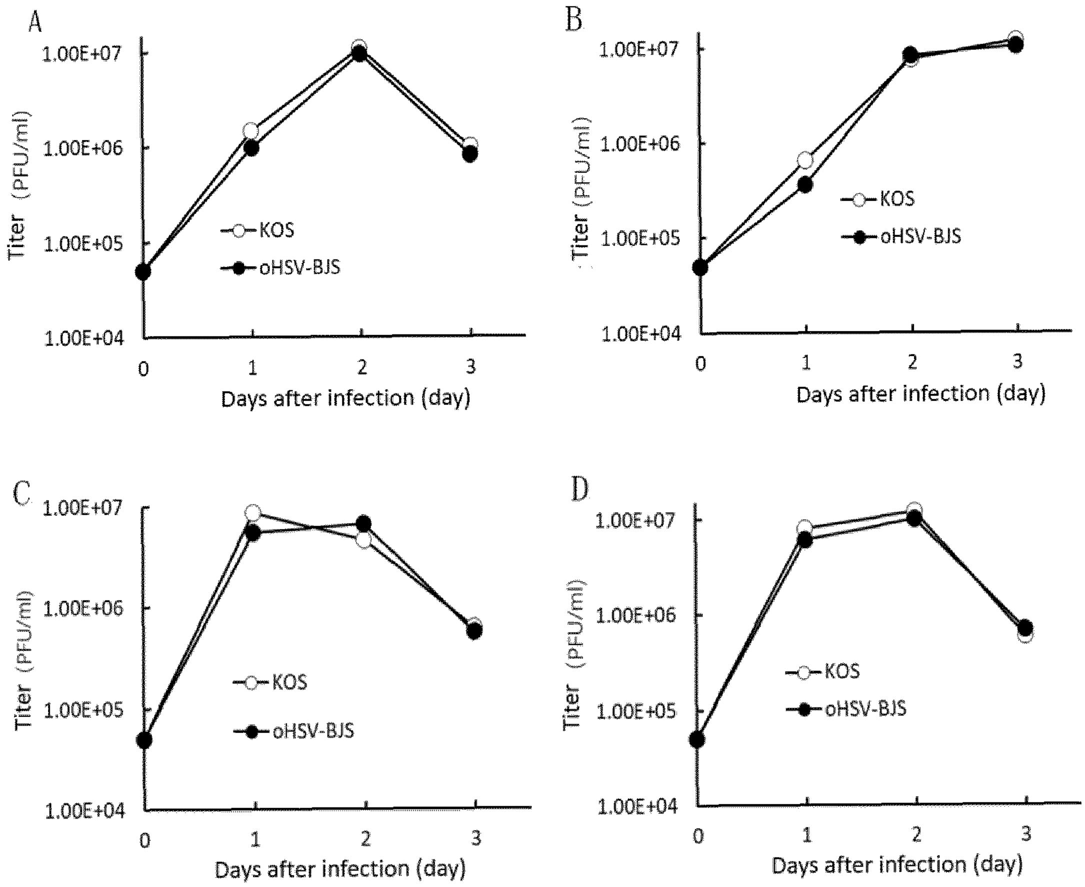
A
1.00E+07
1.00E+06
1.00E+05
1.00E+04
Titer (PFU/ml)
KOS
oHSV-BJS
0
1
2
3
Days after infection (day)
B
C
D

Figure. 5

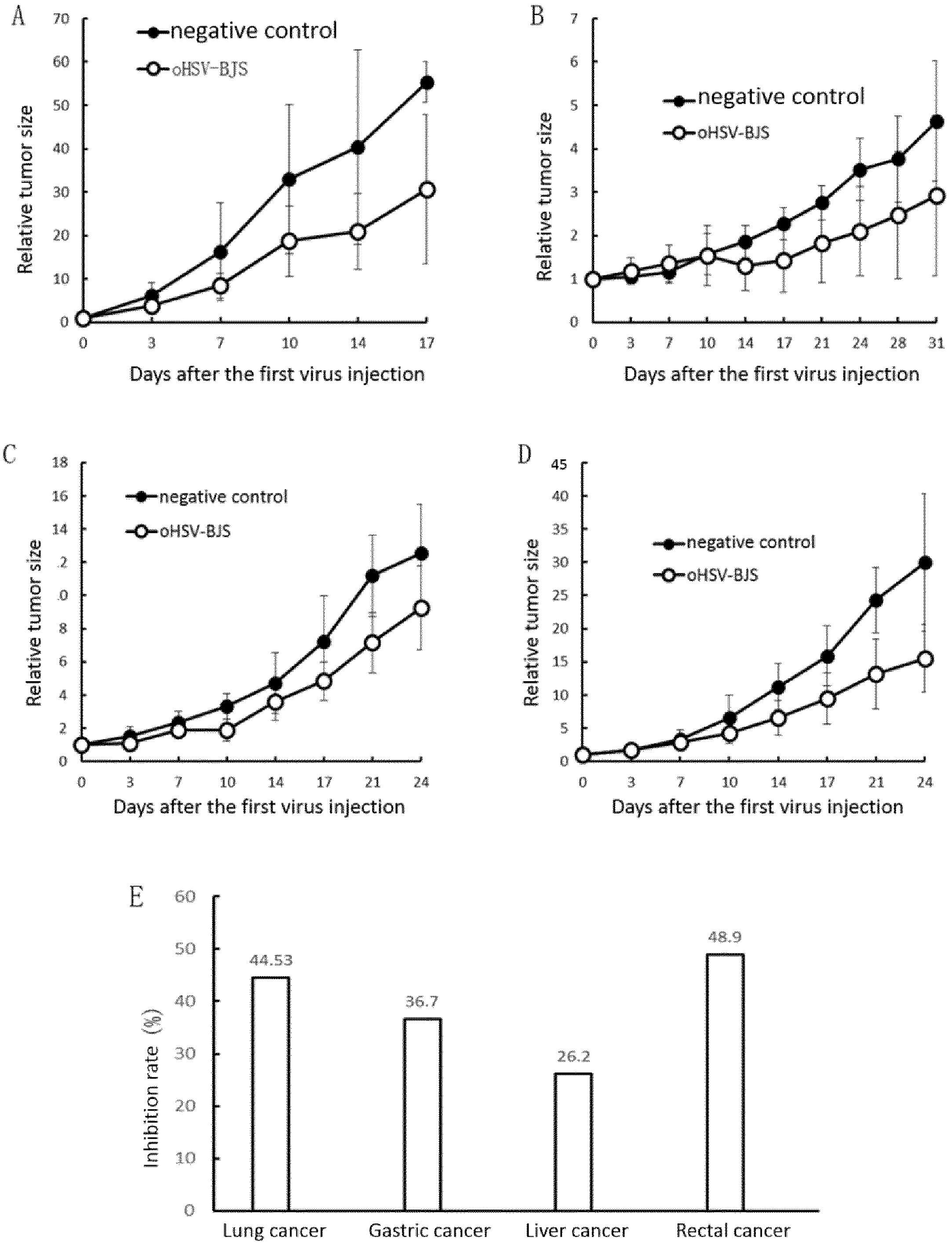
A
negative control
oHSV-BJS
Relative tumor size
Days after the first virus injection
B
negative control
oHSV-BJS
Relative tumor size
Days after the first virus injection
C
negative control
oHSV-BJS
Relative tumor size
Days after the first virus injection
D
negative control
oHSV-BJS
Relative tumor size
Days after the first virus injection
E
Inhibition rate (%)
44.53
36.7
26.2
48.9
Lung cancer
Gastric cancer
Liver cancer
Rectal cancer

Figure. 6

**INTERNATIONAL SEARCH REPORT**

International application No.
**PCT/CN2020/094751**

**A. CLASSIFICATION OF SUBJECT MATTER**

C12N 7/01(2006.01)i; C12N 15/57(2006.01)i; A61K 35/763(2015.01)i; A61K 35/768(2015.01)i; A61P 35/00(2006.01)i

According to International Patent Classification (IPC) or to both national classification and IPC

**B. FIELDS SEARCHED**

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, USTXT, EPTXT, PUBMED, ISI WEB OF SCIENCE, CNKI, 百度学术: 启动子, 信号肽, 蛋白酶, 切割位点, 溶瘤病毒, 肿瘤特异, promoter, signal peptide, protease, cleavage site, oncolytic virus, cancer specific; GENBANK+EMBL-EBI+中国专利生物序列检索平台: search for SEQ ID NO.4-9.

**C. DOCUMENTS CONSIDERED TO BE RELEVANT**

| Category* | Citation of document, with indication, where appropriate, of the relevant passages | Relevant to claim No. |
|---|---|---|
| PX | CN 110669740 A (WU, Zetang) 10 January 2020 (2020-01-10)<br>see claims 1-16 | 1-20 |
| A | CN 109943538 A (BEIJING LINGKE BIOTECHNOLOGY CO., LTD.) 28 June 2019 (2019-06-28)<br>see abstract | 1-20 |
| A | CN 1884556 A (JIANGSU SHUNTANG BIOENGINEERING CO., LTD.) 27 December 2006 (2006-12-27)<br>see entire document | 1-20 |
| A | CN 102051358 A (FUDAN UNIVERSITY) 11 May 2011 (2011-05-11)<br>see entire document | 1-20 |
| A | US 2011034368 A1 (ABBOTT LAB) 10 February 2011 (2011-02-10)<br>see entire document | 1-20 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 August 2020** | **10 September 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/094751**

**C. DOCUMENTS CONSIDERED TO BE RELEVANT**

| Category* | Citation of document, with indication, where appropriate, of the relevant passages | Relevant to claim No. |
|---|---|---|
| A | CAI Z et al. "Targeting strategies of adenovirus-mediated gene therapy and virotherapy for prostate cancer" *Molecular medicine reports,* Vol. 16, No. 5, 30 November 2017 (2017-11-30), ISSN: 1791-3004, pp. 6443-6458, see entire document | 1-20 |

Form PCT/ISA/210 (second sheet) (January 2015)

**INTERNATIONAL SEARCH REPORT**

International application No.
**PCT/CN2020/094751**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2020/094751**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **21**
because they relate to subject matter not required to be searched by this Authority, namely:

[1] The subject matter of claim 21 is "a method for treating a disease in a patient". This technical solution is a treatment method for cancer in a human or animal body, and belongs to subject matter which does not require an international searching authority to perform a search, as stipulated by PCT Rule 39.1.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/094751**

| Patent document cited in search report | Publication date (day/month/year) | Patent family member(s) | Publication date (day/month/year) |
|---|---|---|---|
| CN 110669740 A | 10 January 2020 | None | |
| CN 109943538 A | 28 June 2019 | None | |
| CN 1884556 A | 27 December 2006 | CN 100582232 C | 20 January 2010 |
| CN 102051358 A | 11 May 2011 | CN 102051358 B | 22 August 2012 |
| US 2011034368 A1 | 10 February 2011 | EP 2468881 A3 | 15 August 2012 |
| | | NO 20080882 A | 21 April 2008 |
| | | SG 164383 A1 | 29 September 2010 |
| | | KR 20080031024 A | 07 April 2008 |
| | | MX 2008000985 A | 07 April 2008 |
| | | CA 2615983 A1 | 01 February 2007 |
| | | CN 101595228 A | 02 December 2009 |
| | | EP 2468768 A2 | 27 June 2012 |
| | | JP 2009508470 A | 05 March 2009 |
| | | EP 2468768 A3 | 31 October 2012 |
| | | EP 2484774 A2 | 08 August 2012 |
| | | TW 200745332 A | 16 December 2007 |
| | | EP 2484774 A3 | 14 November 2012 |
| | | ZA 200800207 A | 28 January 2009 |
| | | US 2007065912 A1 | 22 March 2007 |
| | | ZA 200800207 B | 28 January 2009 |
| | | RU 2008106611 A | 27 August 2009 |
| | | EP 1910550 A2 | 16 April 2008 |
| | | AU 2006272706 A1 | 01 February 2007 |
| | | WO 2007014162 A2 | 01 February 2007 |
| | | EP 1910550 A4 | 04 November 2009 |
| | | IL 188841 D0 | 13 April 2008 |
| | | JP 2012235782 A | 06 December 2012 |
| | | US 2013243789 A1 | 19 September 2013 |
| | | WO 2007014162 A3 | 07 May 2009 |
| | | BR PI0613784 A2 | 01 February 2011 |
| | | EP 2468881 A2 | 27 June 2012 |
| | | NO 20080882 L | 21 April 2008 |
| | | RU 2478709 C2 | 10 April 2013 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2019106428044

Sequence Listing

<110>  ZETANG WU

<120>  Oncolytic Virus and Application Thereof, and Drug for Treating Cancer

<130>  OPI21301526EPO

<150>  CN201910642804.4
<151>  2019-07-16

<150>  PCT/CN2020/094751
<151>  2020-06-05

<160>  10

<170>  PatentIn version 3.5

<210>  1
<211>  2679
<212>  DNA
<213>  Artificial sequence

<220>
<223>  The first nucleic acid fragment

<400>  1
aaataactcg agctgcgggc gctgttgttc catcatcctg tcgggcatcg caatgcgatt      60
gtgttatatc gccgtggtgg ccggggtggt gctcgtggcg cttcactacg agcaggagat      120
ccagaggcgc ctgtttgatg tatgacgtca catccaggcc ggcggaaacc ggaacggcat      180
atgcaaactg gaaactgtcc tgtcttgggg cccacccacc cgacgcgtca tatgtaaatg      240
aaaatcgttc ccccgaggcc atgtgtagcc tggatcccaa cgacccgcc catgggtccc      300
aattggccgt cccgttacca agaccaaccc agccagcgta tccacccccg cccgggtccc      360
cgcggaagcg gaacggtgta tgtgatatgc taattaaata catgccacgt acttatggtg      420
tctgattggt ccttgtctgt gccggaggtg gggcgggggc cccgcccggg gggcggaact      480
aggaggggtt tgggagagcc ggccccggca ccacgggtat aaggacatcc accacccggc      540
cggtggtggt gtgcagccgt gttccaacca cggtcgttga cattgattat tgactagtta      600
ttaatagtaa tcaattacgg ggtcattagt tcatagccca tatatggagt tccgcgttac      660
ataacttacg gtaaatggcc cgcctggctg accgcccaac gacccccgcc cattgacgtc      720
aataatgacg tatgttccca tagtaacgcc aatagggact ttccattgac gtcaatgggt      780
ggactattta cggtaaactg cccacttggc agtacatcaa gtgtatcata tgccaagtac      840
gccccctatt gacgtcaatg acggtaaatg gcccgcctgg cattatgccc agtacatgac      900
cttatgggac tttcctactt ggcagtacat ctacgtatta gtcatcgcta ttaccatggt      960
gatgcggttt tggcagtaca tcaatgggcg tggatagcgg tttgactcac ggggatttcc      1020
aagtctccac cccattgacg tcaatgggag tttgttttgg caccaaaatc aacgggactt      1080
tccaaaatgt cgtaacaact ccgccccatt gacgcaaatg ggcggtaggc gtgtacggtg      1140
ggaggtctat ataagcagag ctctctggct aactgccacc atggtgagca agggcgagga      1200
gctgttcacc ggggtggtgc ccatcctggt cgagctggac ggcgacgtaa acggccacaa      1260
gttcagcgtg tccggcgagg gcgagggcga tgccacctac ggcaagctga ccctgaagtt      1320
catctgcacc accggcaagc tgcccgtgcc ctggcccacc ctcgtgacca ccctgaccta      1380
cggcgtgcag tgcttcagcc gctaccccga ccacatgaag cagcacgact tcttcaagtc      1440
cgccatgccc gaaggctacg tccaggagcg caccatcttc ttcaaggacg acggcaacta      1500
caagacccgc gccgaggtga agttcgaggg cgacaccctg gtgaaccgca tcgagctgaa      1560
gggcatcgac ttcaaggagg acggcaacat cctggggcac aagctggagt acaactacaa      1620
cagccacaac gtctatatca tggccgacaa gcagaagaac ggcatcaagg tgaacttcaa      1680
gatccgccac aacatcgagg acggcagcgt gcagctcgcc gaccactacc agcagaacac      1740

```
ccccatcggc gacggccccg tgctgctgcc cgacaaccac tacctgagca cccagtccgc     1800
cctgagcaaa gaccccaacg agaagcgcga tcacatggtc ctgctggagt tcgtgaccgc     1860
cgccgggatc actctcggca tggacgagct gtacaagtaa cgggcctcga ctgtgccttc     1920
tagttgccag ccatctgttg tttgcccctc ccccgtgcct tccttgaccc tggaaggtgc     1980
cactcccact gtcctttcct aataaaatga ggaaattgca tcgcattgtc tgagtaggtg     2040
tcattctatt ctggggggtg gggtggggca ggacagcaag ggggaggatt gggaagacaa     2100
tagcaggcat gctggggatg cggtgggctc tatggcttct gaggtacaat aaaaacaaaa     2160
catttcaaac aaatcgcccc acgtgttgtc cttctttgct catggccggc ggggcgtggg     2220
tcacggcaga tggcgggggt gggcccggcg tacggcctgg gtgggcggag ggaactaacc     2280
caacgtataa atccgtcccc gctccaaggc cggtgtcata gtgcccttag gagcttcccg     2340
cccgggcgca tcccccccttt tgcactatga cagcgacccc cctcaccaac ctgttcttac     2400
gggccccgga cataacccac gtggcccccc cttactgcct caacgccacc tggcaggccg     2460
aaacggccat gcacaccagc aaaacggact ccgcttgcgt ggccgtgcgg agttacctgg     2520
tccgcgcctc ctgtgagacc agcggcacaa tccactgctt tttctttgcg gtatacaagg     2580
acacccacca taccccctccg ctgattaccg agctccgcaa ctttgcggac ctggttaacc     2640
acccgccggt cctacgcgaa ctggtcgaag ctttttata                            2679
```

<210>  2
<211>  3285
<212>  DNA
<213>  Artificial sequence

<220>
<223>  The second nucleic acid fragment

<400>  2

```
ctcgagctgc gggcgctgtt gttccatcat cctgtcgggc atcgcaatgc gattgtgtta       60
tatcgccgtg gtggccgggg tggtgctcgt ggcgcttcac tacgagcagg agatccagag      120
gcgcctgttt gatgtatgac gtcacatcca ggccggcgga aaccggaacg gcatatgcaa      180
actggaaact gtcctgtctt ggggcccacc cacccgacgc gtcatatgta aatgaaaatc      240
gttcccccga ggccatgtgt agcctggatc ccaacgaccc cgcccatggg tcccaattgg      300
ccgtcccgtt accaagacca acccagccag cgtatccacc cccgcccggg tccccgcgga      360
agcggaacgg tgtatgtgat atgctaatta aatacatgcc acgtacttat ggtgtctgat      420
tggtccttgt ctgtgccgga ggtggggcgg gggccccgcc cggggggcgg aactaggagg      480
ggtttgggag agccggcccc ggcaccacgg gtataaggac atccaccacc cggccggtgg      540
tggtgtgcag ccgtgttcca accacggtca cgcttcggtg cctctccccg attcgggccc      600
ggtcgcttgc taccggtgcg ccaccaccag aggccatatc cgacacccca gccccgacgg      660
cagccgacag cccggtcatg gccttgacct ttgctttact ggtggccctc ctggtgctca      720
gctgcaagtc aagctgctct gtgggcttag aagttctttt tcaaggtcct gcgactgaca      780
ttgatatgct aattgacctc ggcctggacc tctccgacag cgatctggac gaggaccccc      840
ccgagccggc ggagagccgc cgcgacgacc tggaatcgga cagcaacggg gagtgttcct      900
cgtcggacga ggacatggaa gacccccacg gagaggacgg accggagccg atactcgacg      960
ccgctcgccc ggcggtccgc ccgtctcgtc cagaagaccc cggcgtaccc agcacccaga     1020
cgcctcgtcc gacggagcgg cagggcccca acgatcctca accagcgccc cacagtgtgt     1080
ggtcgcgcct cggggcccgg cgaccgtctt gctcccccga gcggcacggg ggcaaggtgg     1140
cccgcctcca acccccaccg accaaagccc agcctgcccg cggcggacgc cgtgggcgtc     1200
gcaggggtcg ggtcgcggt ggtcccgggg ccgccgatgg tttgtcggac ccccgccggc     1260
gtgcccccag aaccaatcgc aacccggggg gaccccgccc cggggcgggg tggacggacg     1320
gccccggcgc cccccatggc gaggcgtggc gcggaagtga gcagcccgac ccacccggag     1380
gcccgcggac acggagcgtg cgccaagcac ccccccccgct aatgacgctg gcgattgccc     1440
ccccgcccgc ggacccccgc gccccggccc cggagcgaaa ggcgcccgcc gccgacacca     1500
tcgacgccac cacgcggttg gtcctgcgct ccatctccga gcgcgcggcg gtcgaccgca     1560
tcagcgagag cttcggccgc agcgcacagg tcatgcacga cccctttggg gggcagccgt     1620
ttcccgccgc gaatagcccc tgggccccgg tgctggcggg ccaaggaggg ccctttgacg     1680
ccgagaccag acgggtctcc tgggaaacct tggtcgccca cggcccgagc ctctatcgca     1740
```

```
cttttgccgg caatcctcgg gccgcatcga ccgccaaggc catgcgcgac tgcgtgctgc     1800
gccaagaaaa tttcatcgag gcgctggcct ccgccgacga gacgctggcg tggtgcaaga     1860
tgtgcatcca ccacaacctg ccgctgcgcc cccaggaccc cattatcggg acggccgcgg     1920
cggtgctgga taacctcgcc acgcgcctgc ggccctttct ccagtgctac ctgaaggcgc     1980
gaggcctgtg cggcctggac gaactgtgtt cgcggcggcg tctggcggac attaaggaca     2040
ttgcatcctt cgtgtttgtc attctggcca ggctcgccaa ccgcgtcgag cgtggcgtcg     2100
cggagatcga ctacgcgacc cttggtgtcg ggtcggaga gaagatgcat ttctacctcc      2160
ccggggcctg catggcgggc ctgatcgaaa tcctagacac gcaccgccag gagtgttcga     2220
gtcgtgtctg cgagttgacg gccagtcaca tcgtcgcccc cccgtacgtg cacggcaaat     2280
atttttattg caactccctg ttttagtgat aagatacatt gatgagtttg gacaaaccac     2340
aactagaatg cagtgaaaaa aatgctttat ttgtgaaatt tgtgatgcta ttgctttatt     2400
tgtaaccatt ataagctgca ataaacaagt taacaacaac aattgcattc attttatgtt     2460
tcaggttcag gggaggtgt gggaggtttt ttaaagcaag taaaacctct acaaatgtgg      2520
tatggctgat tatgatcctg caagcctcgt cgtcctggcc ggaccacgct atctgtgcaa     2580
ggtccccggc cccggacgcg cgctccatga gcagagcgcc cgccgccgag gcgaagactc     2640
gggcggcgcc ctgcccgtcc caccaggtca acaggcggta accggcctct tcatcgggaa     2700
tgcgcgcgac cttcagcatc gccggcatgt cccccctggcg gacgggaagt atcaagcttg    2760
tacaataaaa acaaaacatt tcaaacaaat cgccccacgt gttgtccttc tttgctcatg     2820
gccggcgggg cgtgggtcac ggcagatggc gggggtgggc ccggcgtacg gcctgggtgg     2880
gcggagggaa ctaacccaac gtataaatcc gtccccgctc caaggccggt gtcatagtgc     2940
ccttaggagc ttcccgcccg ggcgcatccc cccttttgca ctatgacagc gacccccctc     3000
accaacctgt tcttacgggc cccggacata acccacgtgg cccccccctta ctgcctcaac    3060
gccacctggc aggccgaaac ggccatgcac accagcaaaa cggactccgc ttgcgtggcc     3120
gtgcggagtt acctggtccg cgcctcctgt gagaccagcg gcacaatcca ctgctttttc     3180
tttgcggtat acaaggacac ccaccatacc cctccgctga ttaccgagct ccgcaacttt     3240
gcggacctgg ttaaccaccc gccggtccta cgcgaactgc tcgag                     3285
```

<210> 3
<211> 1550
<212> DNA
<213> Artificial sequence

<220>
<223> The third nucleic acid fragment

<400> 3

```
aagcttgaat tcatggcccc tccctcgggt taccccacag cctaggccga ttcgacctct       60
ctccgctggg gccctcgctg gcgtccctgc accctgggag cgcgagcggc gcgcgggcgg      120
ggaagcgcgg cccagacccc cgggtccgcc cggagcagct gcgctgtcgg ggccaggccg      180
ggctcccagt ggattcgcgg gcacagacgc ccaggaccgc gctccccacg tggcggaggg      240
actggggacc cgggcacccg tcctgcccct tcaccttcca gctccgcctc ctccgcgcgg      300
accccgcccc gtcccgaccc ctcccgggtc cccggcccag ccccctccgg gccctcccag      360
cccctcccct tcctttccgc ggccccgccc tctcctcgcg gcgcgagttt caggcagcgc      420
tgcgtcctgc tgcgcacgtg ggaagccctg gccccggcca cccccgcgta ggcgtgtacg      480
gtgggaggcc tatataagca gagctcgttt agtgaaccgt cagatcgcct ggagccacca      540
tgggaccaaa cacagaattt gcactatccc tgttaaggaa aaacataatg actataacaa      600
cctcaaaggg agagttcaca gggttaggca tacatgatcg tgtctgtgtg atacccacac      660
acgcacagcc tggtgatgat gtactagtga atggtcagaa aattagagtt aaggataagt      720
acaaattagt agatccagag aacattaatc tagagcttac agtgttgact ttagatagaa      780
atgaaaaatt cagagatatc aggggattta tatcagaaga tctagaaggt gtggatgcca      840
ctttggtagt acattcaaat aactttacca acactatctt agaagttggc cctgtaacaa      900
tggcaggact tattaatttg agtagcaccc ccactaacag aatgattcgt tatgattatg      960
caacaaaaac tgggcagtgt ggaggtgtgc tgtgtgctac tggtaagatc tttggtattc     1020
atgttggcgg taatggaaga caaggatttt cagctcaact taaaaaacaa tattttgtag     1080
agaaacaata gtaatgatga taagatacat tgatgagttt ggacaaacca caactagaat     1140
```

```
gcagtgaaaa aaatgcttta tttgtgaaat ttgtgatgct attgctttat ttgtaaccat     1200
tataagctgc aataaacaag ttaacaacaa caattgcatt cattttatgt ttcaggttca     1260
gggggaggtg tgggaggttt tttaaagcaa gtaaaacctc tacaaatgtg gtatggctga     1320
ttatgatcct gcaagcctcg tcgtcctggc cggaccacgc tatctgtgca aggtccccgg     1380
ccccggacgc gcgctccatg agcagagcgc ccgccgccga ggcgaagact cgggcggcgc     1440
cctgcccgtc ccaccaggtc aacaggcggt aaccggcctc ttcatcggga atgcgcgcga     1500
ccttcagcat cgccggcatg tccccctggc ggacgggaag tatcaagctt                 1550


<210>  4
<211>  426
<212>  DNA
<213>  Artificial sequence

<220>
<223>  The nucleotide sequence of the telomerase reverse transcriptase promoter

<400>  4
gaattcatgg cccctccctc gggttacccc acagcctagg ccgattcgac ctctctccgc       60
tggggccctc gctggcgtcc ctgcaccctg ggagcgcgag cggcgcgcgg gcggggaagc      120
gcggcccaga cccccgggtc cgcccggagc agctgcgctg tcggggccag gccgggctcc      180
cagtggattc gcgggcacag acgcccagga ccgcgctccc cacgtggcgg agggactggg      240
gacccgggca cccgtcctgc cccttcacct tccagctccg cctcctccgc gcggaccccg      300
ccccgtcccg acccctcccg ggtccccggc ccagcccccct ccgggccctc ccagcccctc      360
cccttccttt ccgcggcccc gccctctcct cgcggcgcga gtttcaggca gcgctgcgtc      420
ctgctg                                                                 426


<210>  5
<211>  182
<212>  PRT
<213>  Artificial sequence

<220>
<223>  The amino acid sequence of human rhinovirus 3C protease

<400>  5
Gly Pro Asn Thr Glu Phe Ala Leu Ser Leu Leu Arg Lys Asn Ile Met
1               5                   10                  15
Thr Ile Thr Thr Ser Lys Gly Glu Phe Thr Gly Leu Gly Ile His Asp
            20                  25                  30
Arg Val Cys Val Ile Pro Thr His Ala Gln Pro Gly Asp Asp Val Leu
        35                  40                  45
Val Asn Gly Gln Lys Ile Arg Val Lys Asp Lys Tyr Lys Leu Val Asp
    50                  55                  60
Pro Glu Asn Ile Asn Leu Glu Leu Thr Val Leu Thr Leu Asp Arg Asn
65                  70                  75                  80
Glu Lys Phe Arg Asp Ile Arg Gly Phe Ile Ser Glu Asp Leu Glu Gly
                85                  90                  95
Val Asp Ala Thr Leu Val Val His Ser Asn Asn Phe Thr Asn Thr Ile
            100                 105                 110
Leu Glu Val Gly Pro Val Thr Met Ala Gly Leu Ile Asn Leu Ser Ser
        115                 120                 125
Thr Pro Thr Asn Arg Met Ile Arg Tyr Asp Tyr Ala Thr Lys Thr Gly
    130                 135                 140
Gln Cys Gly Gly Val Leu Cys Ala Thr Gly Lys Ile Phe Gly Ile His
```

```
145                 150                 155                 160
Val Gly Gly Asn Gly Arg Gln Gly Phe Ser Ala Gln Leu Lys Lys Gln
                165                 170                 175
Tyr Phe Val Glu Lys Gln
            180


<210>  6
<211>  552
<212>  DNA
<213>  Artificial sequence

<220>
<223>  The nucleotide sequence of the open reading frame of human rhinovirus 3C
protease

<400>  6
atgggaccaa acacagaatt tgcactatcc ctgttaagga aaaacataat gactataaca      60
acctcaaagg gagagttcac agggttaggc atacatgatc gtgtctgtgt gatacccaca     120
cacgcacagc ctggtgatga tgtactagtg aatggtcaga aaattagagt taaggataag     180
tacaaattag tagatccaga gaacattaat ctagagctta cagtgttgac tttagataga     240
aatgaaaaat tcagagatat caggggattt atatcagaag atctagaagg tgtggatgcc     300
actttggtag tacattcaaa taactttacc aacactatct tagaagttgg ccctgtaaca     360
atggcaggac ttattaattt gagtagcacc cccactaaca gaatgattcg ttatgattat     420
gcaacaaaaa ctgggcagtg tggaggtgtg ctgtgtgcta ctggtaagat ctttggtatt     480
catgttggcg gtaatggaag acaaggattt tcagctcaac ttaaaaaaca atattttgta     540
gagaaacaat ag                                                         552


<210>  7
<211>  23
<212>  PRT
<213>  Artificial sequence

<220>
<223>  The amino acid sequence of the interferon ¦Á2 signal peptide

<400>  7
Met Ala Leu Thr Phe Ala Leu Leu Val Ala Leu Leu Val Leu Ser Cys
1               5                   10                  15
Lys Ser Ser Cys Ser Val Gly
            20


<210>  8
<211>  69
<212>  DNA
<213>  Artificial sequence

<220>
<223>  The second nucleic acid encoding the interferon ¦Á2 signal peptide

<400>  8
atggccttga cctttgcttt actggtggcc ctcctggtgc tcagctgcaa gtcaagctgc      60
tctgtgggc                                                              69
```

```
<210>  9
<211>  24
<212>  DNA
<213>  Artificial sequence

<220>
<223>  The nucleotide sequence of the third nucleic acid sequence

<400>  9
ttagaagttc tttttcaagg tcct                                            24


<210>  10
<211>  66
<212>  DNA
<213>  Artificial sequence

<220>
<223>  The base sequence of the CMV enhancer

<400>  10
gtaggcgtgt acggtgggag gcctatataa gcagagctcg tttagtgaac cgtcagatcg      60
cctgga                                                                66
```